(19) Europäisches Patentamt / European Patent Office / Office européen des brevets

(11) **EP 3 395 370 B1**

(12) **EUROPEAN PATENT SPECIFICATION**

(45) Date of publication and mention of the grant of the patent:
**20.01.2021 Bulletin 2021/03**

(21) Application number: **16878661.4**

(22) Date of filing: **20.12.2016**

(51) Int Cl.:
*A61K 47/24* (2006.01)   *A61K 9/127* (2006.01)
*A61K 45/00* (2006.01)   *A61K 47/28* (2006.01)
*A61K 47/32* (2006.01)   *A61K 47/34* (2017.01)
*A61P 35/00* (2006.01)   *A61K 31/704* (2006.01)
*A61K 9/00* (2006.01)

(86) International application number:
**PCT/JP2016/087875**

(87) International publication number:
**WO 2017/110772 (29.06.2017 Gazette 2017/26)**

(54) **LIPOSOME AND LIPOSOME COMPOSITION**

LIPOSOM UND LIPOSOMZUSAMMENSETZUNG

LIPOSOMES ET COMPOSITION DE LIPOSOMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2015 JP 2015248337**

(43) Date of publication of application:
**31.10.2018 Bulletin 2018/44**

(73) Proprietor: **FUJIFILM Corporation Tokyo 106-8620 (JP)**

(72) Inventors:
• **YAMADA, Naoki**
  **Ashigarakami-gun**
  **Kanagawa 258-8577 (JP)**
• **MAKITA, Keiko**
  **Ashigarakami-gun**
  **Kanagawa 258-8577 (JP)**
• **WATANABE, Eriko**
  **Ashigarakami-gun**
  **Kanagawa 258-8577 (JP)**
• **INOUE, Takuya**
  **Ashigarakami-gun**
  **Kanagawa 258-8577 (JP)**

(74) Representative: **Hoffmann Eitle Patent- und Rechtsanwälte PartmbB Arabellastraße 30 81925 München (DE)**

(56) References cited:
EP-A1- 2 253 308       WO-A1-96/10392
WO-A1-2008/028917    WO-A1-2015/135441
JP-A- H10 506 395       JP-A- 2010 502 671
JP-A- 2010 529 191

## Description

Field of the Invention

The present invention relates to a liposome and a liposome composition. The present invention relates to a liposome and a liposome composition, as defined by the claims, which have blood kinetics, suitable as pharmaceuticals such as anticancer agents.

Related Art

Liposomes (also referred to as lipid vesicles) have been studied for a variety of applications such as immunological sensors, artificial erythrocytes, and carriers of a drug delivery system by making use of characteristics such as barrier ability, compound retention ability, biocompatibility, the degree of freedom in setting a particle diameter, readily degradable properties, and surface modification properties. In the application of carriers of a drug delivery system, liposomes can contain water-soluble compounds, lipid-soluble low molecules, polymers, and a wide range of substances.

A liposome of which the surface is modified with a hydrophilic polymer represented by polyethylene glycol (PEG) can escape from capturing of the reticuloendothelial system and have high retentivity in blood. As a result, in a case of a particle size of about less than or equal to 400 nm, high accumulation properties in tumors and inflamed lesions are exhibited by an enhanced permeability and retention (EPR) effect. The EPR effect means a phenomenon (enhanced permeability) in which there is a gap between vascular endothelial cells in a lesion site such as a tumor or inflammation and liposomes tend to be accumulated in normal tissues, and a phenomenon (enhanced retention) in which development of the lymphatic system in a lesion site is immature, and therefore, accumulated liposomes are not excluded and further accumulation in the lesion site proceeds (Non-Patent Document 1)

In a case where a liposome formulation of which the surface is modified with a hydrophilic polymer to extend retention in blood using the EPR effect is applied to a tumor disease, since a drug is contained in a liposome, it is possible to expect that drug exposure may be enhanced by increasing accumulation of liposomes in a tumor while reducing the exposure of the drug to the cardiovascular system or the like, thereby improving a treatment index. According to the principle described above, DOXIL (registered trademark) achieves drug efficacy and reduction in cardiotoxicity due to accumulation of DOXIL in tumors, and is widely used as a commercially available medicine containing doxorubicin.

Patent Document 1 discloses a liposome composition including: a liposome which has a sufficient size for extravasation into a tumor and is formed of a vesicle-forming lipid and 1 to 20 mol% of an amphipathic vesicle-forming lipid derivatized with a hydrophilic polymer selected from polyethylene glycol, polylactic acid, polyglycolic acid, and a polylactic acid-polyglycolic acid copolymer; and a tumor imaging agent or an antitumor agent in a form of being incorporated into the liposome, as a liposome composition in which the EPR effect is utilized. WO 96/10392 relates to liposomal preparations composed of DOPE (dioleyl phosphatidylethanolamine) and POPS (palmitoyl-oleyl phosphatidylserine) wherein DMPE-PEG2000 was added.

Prior Art Documents

Patent Document

Patent Document 1: JP2889549B

Non-Patent Document

Non-Patent Document 1: Cancer Research, (1986), 46, pp. 6387-6392).

## SUMMARY OF THE INVENTION

On the other hand, in some cases, accumulation of liposomes in normal tissues may be enhanced due to the EPR effect, thereby causing serious side effects. That is, in some cases, in treatment of administering DOXIL (registered trademark), side effects, such as appearance of erythema, pigmentation, and blisters, which are called hand-foot syndromes and accompanied by strong pain may occur in sites, such as hands or feet or the vicinity of the waist on which a belt is attached, where an ordinary stimulus is caused, and therefore, the treatment has to be discontinued (Arch Dermatol. 2000; (136), pp. 1475-1780). It is considered that this is because liposomes are accumulated in an inflamed area of the skin due to the EPR effect.

On the other hand, the above-described side effects do not occur in a commercially available doxorubicin-containing liposome Myocet (registered trademark) which is rapidly released and has a short retention time in blood which is a liposome formulation containing doxorubicin similarly to DOXIL (registered trademark). Accordingly, it is considered that the side effects of the above-described hand-foot syndrome occur because retention of DOXIL (registered trademark) of which the retention period is too long in blood causes adverse effects.

An object of the present invention is to provide a liposome and a liposome composition in which release of a drug in blood is suppressed during accumulation of the liposome and the liposome composition in a target site such as a tumor and the drug is rapidly removed after the liposome and the liposome composition are sufficiently accumulated.

The present inventors have conducted extensive studies in order to solve the above-described problems, and as a result, they have found that it is possible to provide a liposome and a liposome composition of which the above-described problems are solved by achieving optimization of a structure of lipid components forming the liposome, and have completed the present invention.

According to the present invention, the following inventions are provided.

[1] A liposome as defined by the claims, containing at least a phospholipid to which a hydrophilic polymer is bonded, a phospholipid having a negative charge within a range of a pH of 6 to 8, and a phospholipid which is electrically neutral within a range of a pH of 6 to 8, in which an average carbon chain length of the above-described phospholipid to which a hydrophilic polymer is bonded is shorter than an average carbon chain length of the above-described phospholipid which is electrically neutral within a range of a pH of 6 to 8.

[2] The liposome according to [1], in which the above-described hydrophilic polymer is polyethylene glycol as defined by the claims,

[3] The liposome according to [1] or [2], in which a weight-average molecular weight of the above-described hydrophilic polymer is 500 to 10,000 as defined by the claims.

[4] The liposome according to any one of [1] to [3], in which the above-described phospholipid to which a hydrophilic polymer is bonded has a negative charge within a range of a pH of 6 to 8.

[5] The liposome according to any one of [1] to [4], as defined by the claims in which the phospholipid to which a hydrophilic polymer is bonded is diacylphosphatidylethanolamine to which a hydrophilic polymer is bonded or diacylglycerol to which a hydrophilic polymer is bonded.

[6] The liposome according to any one of [1] to [5], in which the above-described phospholipid having a negative charge within a range of a pH of 6 to 8 is one or more types selected from the group consisting of phosphatidic acid, phosphatidylglycerol, phosphatidylserine, and phosphatidylinositol.

[7] The liposome according to any one of [1] to [6], in which the above-described phospholipid which is electrically neutral within a range of a pH of 6 to 8 is phosphatidylcholine as defined by the claims.

[8] The liposome according to any one of [1] to [7], in which an average carbon chain length of the phospholipid which is electrically neutral within a range of a pH of 6 to 8 is 16-18 as defined by the claims.

[9] The liposome according to any one of [1] to [8], in which the above-described phospholipid to which a hydrophilic polymer is bonded is dipalmitoyl phosphatidylethanolamine to which polyethylene glycol is bonded or dimyristoyl phosphatidylethanolamine to which polyethylene glycol is bonded, as defined by the claims, and the above-described phospholipid which is electrically neutral within a range of a pH of 6 to 8 is hydrogenated soybean phosphatidylcholine.

[10] The liposome according to any one of [1] to [9], in which a content ratio of the above-described phospholipid to which a hydrophilic polymer is bonded with respect to all lipids forming a liposome membrane is 1 to 20 mol%, a content ratio of the above-described phospholipid having a negative charge within a range of a pH of 6 to 8 with respect to all the lipids forming the liposome membrane is 1 to 20 mol%, and a content ratio of the above-described phospholipid which is electrically neutral within a range of a pH of 6 to 8 with respect to all the lipids forming the liposome membrane is 30 to 80 mol%.

[11] The liposome according to any one of [1] to [10], further comprising: one or more other types of lipids in addition to the above-described phospholipid to which a hydrophilic polymer is bonded, the above-described phospholipid having a negative charge within a range of a pH of 6 to 8, and the above-described phospholipid which is electrically neutral within a range of a pH of 6 to 8.

[12] The liposome according to any one of [1] to [11], in which the other types of the above-described lipids are cholesterols.

[13] The liposome according to any one of [1] to [12] which is dispersed in a medium which disperses the liposome.

[14] A liposome composition comprising: the liposome according to any one of [1] to [13]; and a drug contained in the above-described liposome.

[15] The liposome composition according to [14], in which the above-described drug has a distribution coefficient LogD of less than or equal to 1.

According to the present invention, there are provided a liposome and a liposome composition in which release of a drug in blood is suppressed during accumulation of the liposome and the liposome composition in a target site such as a tumor and the drug is rapidly removed after the liposome and the liposome composition are sufficiently accumulated.

## BRIEF DESCRIPTION OF THE DRAWINGS

Fig. 1 shows results of an evaluation of blood kinetics for a doxorubicin solution and liposome compositions of Examples 1 and 2 and Comparative Examples 1 and 3.
Fig. 2 shows results of an evaluation of blood kinetics for a doxorubicin solution and liposome compositions of Example 2 and Comparative Examples 1 and 3.
Fig. 3 shows results of an evaluation of blood kinetics for liposome compositions of Comparative Examples 1 and 2.
Fig. 4 shows results of an evaluation of blood kinetics for liposome compositions of Comparative Examples 1 and 4.
Fig. 5 shows results of drug efficacy tests for liposome compositions of Example 1 and Comparative Example 1, a doxorubicin solution, and a reference solvent.
Fig. 6 shows results of drug efficacy tests for liposome compositions of Comparative Examples 1 and 2 and a reference solvent.
Fig. 7 shows results of drug efficacy tests for liposome compositions of Comparative Examples 1 and 4 and a reference solvent.

## DESCRIPTION OF THE PREFERRED EMBODIMENTS

In the present specification, the term "step" includes not only an independent step, but also a case where an intended purpose of the step is achieved even in a case where the step cannot be clearly distinguished from other steps.

In the present specification, the numerical range represented by "to" indicates a range including numerical values denoted before and after "to" as a minimum value and a maximum value.

In the present specification, the amount of each component in a composition means, unless otherwise specified, a total amount of a plurality of substances present in liposome or the composition in a case where the plurality of substances corresponding to each component are present in liposome or the composition.

The "release" means that a drug encapsulated in a liposome passes through a lipid membrane forming the liposome and is released to the outside of the liposome.

"Retentivity in blood" means a property (state) in which a drug in a state of being encapsulated in a liposome is present in blood in a target to which a liposome (or liposome composition) is administered.

The "average particle diameter of the liposome" means a volume average particle diameter of liposomes. The average particle diameter of liposomes is measured through a dynamic light scattering method. Examples of commercially available determination devices using dynamic light scattering include a concentrated particle analyzer FPAR-1000 (manufactured by OTSUKA ELECTRONICS Co., LTD.), NANOTRAC UPA (manufactured by Nikkiso Co., Ltd.), and NANOSIZER (manufactured by Malvern Instruments Ltd.)

The "target" is a mammal, such as a human, a mouse, a monkey, or a domestic animal, requiring prevention or treatment of a tumor, and is preferably a human who requires prevention or treatment thereof.

Specific examples of the "tumor" (used synonymously with "cancer" in the present invention) include solid tumors such as esophageal cancer, stomach cancer (including gastric adenocarcinoma), colorectal cancer, colon cancer, rectal cancer, liver cancer, pancreatic cancer, renal cell carcinoma, laryngeal cancer, lung cancer (including non-small-cell lung cancer), prostate cancer, bladder cancer, breast cancer, uterine cancer (including endometrial cancer), ovarian cancer, urothelial cancer, head and neck squamous cell cancer, Kaposi's sarcoma, melanoma, and non-Hodgkin lymphoma, and humoral tumors such as leukemia. The sites where tumors develop are the cells, tissues, and organs of tumors, and the inside of the cells, the tissues, and the organs.

Hereinafter, the present invention will be described in detail.

The present invention relates to a liposome as defined by the claims, containing at least a phospholipid to which a hydrophilic polymer is bonded, a phospholipid having a negative charge within a range of a pH of 6 to 8, and a phospholipid which is electrically neutral within a range of a pH of 6 to 8, in which an average carbon chain length of the phospholipid to which a hydrophilic polymer is bonded is shorter than an average carbon chain length of the phospholipid which is electrically neutral within a range of a pH of 6 to 8.

A liposome formed of the phospholipid to which a hydrophilic polymer is bonded exhibits high retentivity in blood and can be highly accumulated in a lesion part such as a tumor due to the EPR effect. However, in some cases, the above-described liposome causes hand-foot syndrome as a new side effect, and therefore, it is difficult to continue the administration. It is considered that, in a case where the retention time of the liposome in blood is too long, the hand-foot syndrome is caused by inflammation of the skin due to the drug contained in the liposome and by accumulation of the liposomes due to the EPR effect in the next administration. In the present invention, it has been found that it is possible to achieve excellent in vivo kinetics, for example, inflammation is not caused on the skin while making a liposome stay in blood only for a sufficient period to be accumulated in a tumor, by forming the liposome using the phospholipid which is electrically neutral within a range of a pH of 6 to 8 in addition to the phospholipid to which a hydrophilic polymer is bonded and the phospholipid having a negative charge within a range of a pH of 6 to 8 and further making an average carbon chain length of the phospholipid to which a hydrophilic polymer is bonded be shorter than an average carbon chain length of the phospholipid which is electrically neutral within a range of a pH of 6 to 8. Particularly, the fact that it is possible to achieve the above-described excellent in vivo kinetics using a characteristic of the combination of the phospholipid to which a hydrophilic polymer is bonded, the phospholipid having a negative charge within a range of a pH of 6 to 8, and the phospholipid which is electrically neutral within a range of a pH of 6 to 8 and a characteristic that the average carbon chain length of the phospholipid to which a hydrophilic polymer is bonded is shorter than the average carbon chain length of the phospholipid which is electrically neutral within a range of a pH of 6 to 8 is absolutely unexpected. In addition, the liposome of the present invention exhibits the excellent in vivo kinetics described above, thereby exhibiting sufficient drug efficacy and at the same time reducing side effects. As a mechanism capable of achieving the excellent blood kinetics described above, it is presumed as follows, but the present invention is not limited by the following mechanism.

In a case where the average carbon chain length of the phospholipid to which a hydrophilic polymer is bonded is shorter than the average carbon chain length of the phospholipid which is electrically neutral within a range of a pH of 6 to 8, the phospholipid to which a hydrophilic polymer is bonded easily falls off from a lipid bilayer forming the liposome. As a result, it is possible to suppress the inflammation of the skin using the liposomes remaining in blood until a certain period of time sufficient to be accumulated in the tumor and by promoting exclusion of the liposome particles from the blood before the accumulation of the liposomes on the skin progresses. However, in a case where the amount of negative charge on the surfaces of the liposome particles after the falling off of the hydrophilic polymer is small, it is considered that, in some cases, accumulation of the liposomes in a specific organ is promoted, which may cause toxicity in various ways.

In addition, in a case where electric charges of the liposomes from which the phospholipid to which a hydrophilic polymer is bonded falls off are negative charges due to the presence of the phospholipid having a negative charge within a range of a pH of 6 to 8, it is possible to reduce the toxicity.

With such a configuration of the present invention, it is possible to presume that the development of unexpected drug efficacy and reduction in side effects are realized.

[Liposome]

A liposome is a closed endoplasmic reticulum formed of a lipid bilayer membrane using a lipid and has an aqueous phase (inner aqueous phase) in a space of the closed endoplasmic reticulum. The inner aqueous phase contains water and the like. Liposomes usually exist in a state of being dispersed in an aqueous solution (outer aqueous phase) on the outside of the closed endoplasmic reticulum. A liposome may be a single lamella (also called a monolayer lamella or a unilamella, with a single layer structure of a bilayer membrane), or may be a multilayer lamella (also called a multilamella with a structure of multiple bilayer membranes having an onion shape in which each of the layers is partitioned by a watery layer). However, a liposome of a single lamella is preferable in the present invention from the viewpoints of safety and stability in medical use.

The form of the liposome is not particularly limited as long as it is a liposome capable of containing a drug. The "containing" means a form in which a drug is included in an inner aqueous phase of the liposome. For example, a form in which a drug is contained in a closed space formed of a membrane, a form in which a drug is contained in a membrane itself, or a combination thereof may be used.

The average particle diameter of liposomes is not particularly limited, but it is 10 to 200 nm, preferably 15 to 150 nm, and more preferably 20 to 120 nm.

Components (membrane components) forming a lipid bilayer of a liposome can be selected from lipids. A lipid dissolving in a mixed solvent of a water-soluble organic solvent and an ester-based organic solvent can be optionally used as the lipids. Particularly in the present invention, the phospholipid to which a hydrophilic polymer is bonded, the phospholipid having a negative charge within a range of a pH of 6 to 8, and the phospholipid which is electrically neutral within a range of a pH of 6 to 8 are used as the lipids, as defined by the claims. In the present invention, "phospholipids" also include phospholipid derivatives modified with phospholipids.

The hydrophilic polymer in the phospholipid to which a hydrophilic polymer is 1 bonded, is polyethylene glycol as defined by the claims.

Polyethylene glycol (PEG) is most preferred because it is versatile and has an effect of improving the retentivity in blood.

The weight-average molecular weight of PEG is either 2000 or 5000 as defined by the claims. Dipalmitoyl phosphatidylethanolamine (DPPE-PEG5000) to which PEG having a weight-average molecular weight of 5,000 is bonded and dimyristoyl phosphatidylethanolamine (DMPE-PEG2000) to which PEG having a weight-average molecular weight of 2,000 is bonded are particularly preferable.

The phospholipid having a negative charge within a range of a pH of 6 to 8 means a lipid having an acidic group from which a proton is separated within a range of a pH of 6 to 8. The phospholipid having a negative charge within a range of a pH of 6 to 8 is not particularly limited, but examples thereof include phosphatidylglycerol, phosphatidic acid, phosphatidylserine, and phosphatidylinositol.

The phospholipid which is electrically neutral within a range of a pH of 6 to 8 means a lipid having no acidic group from which a proton is separated within a range of a pH of 6 to 8 or a basic group to be protonated. The phospholipid (a phospholipid having no electric charge) which is electrically neutral within a range of a pH of 6 to 8 is phosphatidylcholine, phospholipid hydrogenated soybean phosphatidylcholine (HSPC) obtained by hydrogenating the same, and hydrogenated soybean phosphatidylcholine is preferable.

The average carbon chain length of the phospholipid which is electrically neutral within a range of a pH of 6 to 8 is 16 to 18.

The average carbon chain length can be obtained from a value of the carbon chain length of each phospholipid (one or more) used as the phospholipid which is electrically neutral within a range of a pH of 6 to 8 and the content ratio of each phospholipid (the ratio of the mass of each phospholipid to the total mass of the phospholipid which is electrically neutral within a range of a pH of 6 to 8).

Specific examples of the preferred embodiment of the present invention include a case where the phospholipid to which a hydrophilic polymer is bonded is dipalmitoyl phosphatidylethanolamine to which polyethylene glycol is bonded or dimyristoyl phosphatidylethanolamine to which polyethylene glycol is bonded, as defined by the claims, and the phospholipid which is electrically neutral within a range of a pH of 6 to 8 is hydrogenated soybean phosphatidylcholine.

The content ratio of the phospholipid to which a hydrophilic polymer is bonded with respect to the all the lipids forming the liposome membrane is preferably 1 to 20 mol%, more preferably 2 to 15 mol%, still more preferably 2 to 10 mol%, and particularly preferably 3 to 10 mol%.

The content ratio of the phospholipid having a negative charge within a range of a pH of 6 to 8 with respect to the all the lipids forming the liposome membrane is preferably 1 to 20 mol%, more preferably 2 to 15 mol%, still more preferably 2 to 10 mol%, and particularly preferably 3 to 10 mol%.

The content ratio of the phospholipid which is electrically neutral within a range of a pH of 6 to 8 with respect to the all the lipids forming the liposome membrane is preferably 30 to 80 mol%, more preferably 35 to 70 mol%, and still more preferably 40 to 60 mol%.

The liposome of the present invention may further include: one or more other types of lipids in addition to the phospholipid to which a hydrophilic polymer is bonded, the phospholipid having a negative charge within a range of a pH of 6 to 8, and the phospholipid which is electrically neutral within a range of a pH of 6 to 8.

Examples of the above-described other types of lipids include lipids other than phospholipids. Examples of the lipids other than phospholipids include lipids not containing phosphoric acid. The lipids other than phospholipids are not particularly limited, but examples thereof include glycerolipids having no phosphoric acid moiety in its molecule and sphingolipids having no phosphoric acid moiety in its molecule. In the present invention, "lipids other than phospholipids" also include derivatives of lipids other than phospholipids modified with lipids other than phospholipids.

In a case where the lipids other than phospholipids contain a basic functional group, for example, a case of a substance in which a compound having a basic functional group is bonded to a lipid, the lipid is called a cationized lipid. The cationized lipid can modify, for example, a membrane of a liposome, and therefore, it is possible to enhance the adhesiveness and the like to cells that are target sites.

The above-described the other lipids may be cholesterols. Examples of cholesterols include cholesterol, in which cyclopentahydrophenanthrene is a basic skeleton and a part or all of the carbon is hydrogenated, and a derivative thereof. An example thereof includes cholesterol. In a case where the average particle diameter of liposomes is reduced to less than or equal to 100 nm, the curvature of a lipid membrane increases. Since distortion of membranes arranged in liposomes also increases, a water-soluble drug is more likely to leak out. It is effective to add cholesterol or the like in order to fill the distortion of membranes due to lipids (membrane stabilizing effect) as means to suppress the leakage.

It is expected that the addition of cholesterols to liposomes lowers the fluidity of membranes of the liposomes by, for example, filling the gap between the membranes of the liposomes. In a case where the liposome of the present invention contains cholesterols, the content of cholesterols with respect to the total amount of lipids forming the liposome is not particularly limited, but is preferably 10 to 70 mol%, more preferably 20 to 60 mol%, and still more preferably 30 to 50 mol%.

In addition to the above-described components, a hydrophilic polymer or the like for improving retentivity in blood, a fatty acid, diacetyl phosphate, or the like as a stabilizer of a membrane structure, and $\alpha$-tocopherol or the like as an antioxidant may be added to a liposome. In the present invention, it is preferable not to use additives such as surfactants, for example, dispersion assistants which are not permitted for use in intravenous injection in medicinal use.

The liposome of the present invention can be provided in a state of being dispersed in a medium which disperses the liposome. The above-described medium is not particularly limited as long as it is a medium which can disperse the liposome, but is preferably an aqueous medium. The above-described aqueous medium is also referred to as an outer aqueous phase and will be described below in the present specification.

[Liposome Composition]

(Drug)

In a case where a drug is contained in the liposome of the present invention, a liposome composition can be provided. The drug may be any drug that can be contained in liposomes, and specific examples thereof include water-soluble substances such as an enzyme, a protein, a peptide, nucleic acid (DNA, mRNA, small interfering RNA (siRNA), microRNA (miRNA)), a low molecular compound, a saccharide (an oligosaccharide and a polysaccharide), a polymer compound, an antitumor agent, an antibacterial agent, a contrast agent, an antioxidant, an anti-inflammatory agent, a whitening agent, a moisturizer, and a hair growing agent which have a physiological activity or a pharmacological activity. However, the present invention is not limited thereto. In a case of using a liposome as a carrier of a drug delivery system, low molecular compounds are preferable from the viewpoint of stability. The liposome composition of the present invention can be provided as a pharmaceutical composition, a cosmetic composition, a food composition, or the like, but is not particularly limited. A specific example of the pharmaceutical composition includes an antitumor agent, but is not particularly limited.

The drug may be a water-soluble drug or a water-insoluble drug, but is not particularly limited.

A drug having low lipid solubility can be preferably used as the drug. A drug of which the lipid solubility is higher than a predetermined value is dissolved in a lipid forming a liposome membrane and is continued to be distributed to an outer aqueous phase, so that leakage from an inner aqueous phase easily occurs. Accordingly, it is easy to stably hold the drug in the inner aqueous phase of the liposome using a drug of which the lipid solubility is less than or equal to the predetermined value. It is possible to use a distribution coefficient LogD as a measure of the lipid solubility.

LogD in the present invention shows a distribution coefficient between water and 1-octanol which is adjusted to the pH of an inner aqueous phase containing a drug. First, a drug is dissolved in an aqueous phase of which the pH is adjusted using a buffer solution, the same amount of 1-octanol is added thereto, the mixture is sufficiently mixed, and each concentration of the aqueous phase and the 1-octanol phase is measured using a liquid chromatography or the like. Then, the concentration of the aqueous phase is divided by the concentration of the 1-octanol phase and a logarithm is taken to calculate LogD.

In a case of using a drug having LogD of less than or equal to 1 at the pH of the inner aqueous phase, the above-described drug can be suitably contained in a liposome. The pH of an inner aqueous phase that can be normally set is 3 to 11 from the viewpoint of suppression of degradation of lipids. Examples of compounds in which LogD shows less than or equal to 1 within the range of the pH thereof include doxorubicin, daunorubicin, epirubicin, vincristine, vinblastine, fluorouracil, gemcitabine, cytarabine, and pemetrexed, and an example thereof includes doxorubicin.

A drug may be used in a form of a salt.

Examples of salts of drugs include salts of basic groups such as amino groups, and salts of acidic groups such as hydroxyl groups and carboxyl groups, which are commonly known.

Examples of the salts of basic groups include salts of mineral acids such as hydrochloric acid, hydrobromic acid, nitric acid, and sulfuric acid; salts of organic carboxylic acids such as formic acid, acetic acid, citric acid, oxalic acid, fumaric acid, maleic acid, succinic acid, malic acid, tartaric acid, aspartic acid, and trichloroacetic acid and trifluoroacetic acid; and salts of sulfonic acids such as methanesulfonic acid, benzenesulfonic acid, p-toluenesulfonic acid, mesitylenesulfonic acid, and naphthalenesulfonic acid.

Examples of salts of acidic groups include salts of alkali metals such as sodium and potassium; salts of alkaline earth metals such as calcium and magnesium; ammonium salts; and salts of nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

The content of a drug in a liposome composition is not particularly limited, but is preferably 0.1 to 2.0 mg/ml and more preferably 0.2 to 1.0 mg/ml with respect to the liposome composition.

(Other Additives and the Like)

An aqueous solvent, an additive, and the like may be added to the liposome composition of the present invention. The liposome composition may contain at least one of a pharmaceutically acceptable isotonic agent, a stabilizer, an antioxidant, and a pH adjuster in relation to the administration route.

The isotonic agents are not particularly limited, but examples thereof include inorganic salts such as sodium chloride, potassium chloride, sodium hydrogen phosphate, sodium dihydrogen phosphate, and potassium dihydrogen phosphate, polyols such as glycerol, mannitol, and sorbitol, and saccharides such as glucose, fructose, lactose, or sucrose.

The stabilizer is not particularly limited, but examples thereof include saccharides such as glycerol, mannitol, sorbitol, lactose, or sucrose.

The antioxidants are not particularly limited, but examples thereof include ascorbic acid, uric acid, tocopherol homologues (for example, vitamin E or four isomers of tocopherol $\alpha$, $\beta$, $\gamma$, and $\delta$) cysteine, and ethylenediaminetetraacetic acid (EDTA). The above-described stabilizer and antioxidant can be used alone or in combination of two or more thereof.

Examples of pH adjusters include sodium hydroxide, citric acid, acetic acid, triethanolamine, sodium hydrogen phosphate, sodium dihydrogen phosphate, and potassium hydrogen phosphate.

The liposome composition of the present invention may contain a pharmaceutically acceptable organic solvent, collagen, polyvinyl alcohol, polyvinyl pyrrolidone, carboxyvinyl polymer, sodium carboxymethyl cellulose, sodium polyacrylate, sodium alginate, water soluble dextran, sodium carboxymethyl starch, pectin, methyl cellulose, ethyl cellulose, xanthan gum, gum arabic, casein, gelatin, agar, diglycerin, propylene glycol, polyethylene glycol, vaseline, paraffin, stearyl alcohol, stearic acid, human serum albumin (HSA), mannitol, sorbitol, lactose, phosphate-buffered physiological saline (PBS), sodium chloride, saccharides, an in vivo degradable polymer, a serum-free medium, and an additive which is acceptable as a pharmaceutical additive.

In the present invention, it is particularly preferable that the liposome composition contains ammonium sulfate, L-histidine, purified white sugar, sodium hydroxide, hydrochloric acid, and the like.

A container to be filled with the liposome composition is not particularly limited, but it is preferably a material having low oxygen permeability. An example thereof includes a bag using a laminate film having a plastic container, a glass container, an aluminum foil, an aluminum vapor deposition film, an aluminum oxide vapor deposition film, a silicon oxide vapor deposition film, polyvinyl alcohol, an ethylene vinyl alcohol copolymer, polyethylene terephthalate, polyethylene naphthalate, polyvinylidene chloride, and the like as a gas barrier layer. It is possible to shield light by employing a bag as necessary in which colored glass, aluminum foil, aluminum vapor deposition film, or the like is used.

In the container filled with the liposome composition, it is preferable to replace gas in a container space portion and in a drug solution with an inert gas such as nitrogen in order to prevent oxidation caused by oxygen present in the space portion in the container. For example, nitrogen bubbling of an injection solution and filling of the container in a nitrogen atmosphere can be performed.

Parenteral administration is preferable as a method for administering the liposome composition. For example, it is possible to select intravenous injection such as instillation, intramuscular injection, intraperitoneal injection, subcutaneous injection, intraocular injection, and intrathecal injection. A specific example of the method for administering the liposome composition includes administration using a syringe or through instillation.

The dosage of the drug contained in the liposome composition is usually selected within a range of 0.01 mg to 100 mg per kg of body weight per day, but is not particularly limited.

[Method for Producing Liposome and Liposome Composition]

The method for producing a liposome and a liposome composition of the present invention is not particularly limited, but the liposome and the liposome composition of the present invention can be produced, for example, through an emulsification step of emulsifying a lipid dissolved in an organic solvent to form a liposome and a drug loading step of making the liposome obtained in the emulsification method contain a water-soluble drug. The method for producing a liposome and a liposome composition may include other steps such as an evaporation step of evaporating the organic solvent used in the emulsification step as necessary.

In the emulsification step of emulsifying a lipid dissolved in an organic solvent to form a liposome, a liposome may be formed by emulsifying a lipid dissolved in an organic solvent without undergoing a drying and solidifying step. The emulsification step is not particularly limited, but is preferably a step of applying high shearing and forming fine particles in an emulsification step in which an organic solvent is contained. A liposome can be formed by evaporating (removing the solvent) the organic solvent used in the emulsification step as necessary.

(Emulsification Step)

In the emulsification step, an aqueous phase is mixed with an oil phase in which a lipid is dissolved in an organic solvent, and the aqueous solution containing the lipid can be stirred and emulsified. By performing the mixing, stirring, and emulsifying of the aqueous phase and the oil phase in which the lipid is dissolved in the organic solvent, an emulsion in which the oil phase and the aqueous phase are emulsified in an oil-in-water type (O/W type) is prepared. After mixing, a part or all of the organic solvent derived from the oil phase is removed by an evaporation step to be described below to form a liposome. Alternately, a part or all of the organic solvent in the oil phase evaporates in the process of the stirring and the emulsifying to form a liposome.

As the stirring method, ultrasonic wave or mechanical shear force is used for particle miniaturization. In addition, in order to make the particle diameter uniform, an extruder treatment or a microfluidizer treatment can be performed through a filter having a constant pore diameter. Using an extruder or the like, a secondarily formed multivesicular liposome can be released to form a univesicular liposome. In the present invention, from the viewpoint of simplifying the production step, it is preferable to use a liposome in a state in which a drug is not loaded in the next step without being subjected to extrusion treatment.

In the present invention, the average particle diameter of liposomes to be prepared can be controlled by optionally selecting the speed and time of stirring. From the viewpoint of obtaining liposomes having safety and stability, it is preferable to apply shearing to an aqueous solution containing lipids at a circumferential speed of higher than or equal to 20 m/sec. The shearing is not limited. However, specifically, it is preferable to apply shearing at a circumferential speed of 20 m/sec to 35 m/sec and more preferable to apply shearing at a circumferential speed of 23 m/sec to 30 m/sec.

(Oil Phase)

A mixed solvent of a water-soluble organic solvent and an ester-based organic solvent is used as an organic solvent used as an oil phase. In the present invention, it is preferable not to substantially use organic solvents such as chloroform, methylene chloride, hexane, cyclohexane, or the like as organic solvents, and it is more preferable not to use these organic solvents at all.

The water-soluble organic solvent is not particularly limited, but is preferably an organic solvent having a property of being optionally mixed with water. Specific examples of the water-soluble organic solvent include alcohols such as methanol, ethanol, n-propanol, isopropanol, n-butanol, isobutanol, and tert-butanol, glycols such as glycerin, ethylene glycol, and propylene glycol, and polyalkylene glycols such as polyethylene glycol. Among these, alcohols are preferable. As alcohols, at least one selected from ethanol, methanol, 2-propanol, and tert-butanol is preferable, at least one selected from ethanol, 2-propanol, and tert-butanol is more preferable, and ethanol is still more preferable.

The ester-based organic solvent is not particularly limited, but is preferably an ester obtained from a reaction between an organic acid and alcohol. Specifically, the ester-based organic solvent is preferably at least one selected from ethyl acetate, methyl acetate, isopropyl acetate, tert-butyl acetate, and methyl propionate, more preferably ethyl acetate, isopropyl acetate, and methyl propionate, and still more preferably ethyl acetate.

The mixing ratio of the water-soluble organic solvent to the ester-based organic solvent is not particularly limited, but is, by mass ratio, 90:10 to 30:70, preferably 80:20 to 40:60, and more preferably 80:20 to 70:30. The mixed solvent of the water-soluble organic solvent and the ester-based organic solvent may further contain an aqueous solvent such as water or a buffer solution described below. The aqueous solvent may be added within a range of, for example, 1 to 30 mass%. Although the pH of the mixed solvent is not particularly limited, a range of about 3 to 10 is preferable and a range of about 4 to 9 is more preferable. The ester-based organic solvent may contain physiologically active substances such as various drugs soluble in these solvents.

In a case where ethanol is used as the water-soluble organic solvent and ethyl acetate is used as the ester-based organic solvent, the mixing ratio of ethanol to ethyl acetate is not particularly limited, but is preferably 80:20 to 70:30 by mass ratio.

The concentration of the lipid is not particularly limited and can be appropriately adjusted. However, in a solution using a mixed solution of a water-soluble organic solvent and an ester-based organic solvent, as a solvent, the concentration may be 20 g/L to 200 g/L and is preferably 30 g/L to 100 g/L.

(Aqueous Phase)

The aqueous phase means an outer aqueous phase and an inner aqueous phase.

The outer aqueous phase in the present invention means an aqueous solution which disperses liposomes. In a case of injections, for example, a solution occupying the outside of liposomes of a liposome dispersion liquid stored by being packaged in a vial or a prefilled syringe becomes an outer aqueous phase. In addition, regarding a solution dispersed at the time of use during administration using an attached liquid for dispersion or other dissolution liquid, a solution occupying the outside of liposomes of a liposome dispersion liquid becomes an outer aqueous phase.

The inner aqueous phase in the present invention means an aqueous phase in a closed endoplasmic reticulum separated by a lipid bilayer membrane.

In a case of producing liposomes, water (distilled water, water for injection, or the like), physiological saline, various buffer solutions, aqueous solutions of saccharides, and a mixture thereof (aqueous solvent) are preferably used as aqueous solutions (outer aqueous phases) which disperse liposomes. The buffer solution is not limited to an organic type and an inorganic type. However, a buffer solution having a buffering action in the vicinity of the hydrogen ion concentration close to a body fluid is suitably used, and examples thereof include a phosphate buffer solution, a Tris buffer solution, a citrate buffer solution, an acetate buffer solution, and Good's buffer solution. The pH of the aqueous phase is not particularly limited, but may be 5 to 9 and preferably 7 to 8. For example, it is preferable to use a phosphate buffer solution (for example, pH = 7.4). In a case of producing liposomes, an inner aqueous phase of liposomes may be an aqueous solution which disperses liposomes, or water, physiological saline, various buffer solutions, aqueous solutions of saccharides, and a mixture thereof. Water used as an outer aqueous phase or an inner aqueous phase preferably does not contain impurities (such as dust and chemical substances).

The physiological saline means an inorganic salt solution adjusted so as to be isotonic with a human body, and may have a buffering function. Examples of the physiological saline include saline containing 0.9 w/v% sodium chloride, phosphate-buffered physiological saline (also referred to as PBS), and Tris-buffered physiological saline.

(Evaporation Step)

In the present invention, an evaporation step may be provided as necessary. In the evaporation step, an organic solvent is evaporated from an aqueous solution containing the liposomes obtained in the emulsification step. In the present invention, the evaporation step includes at least a step of forcibly removing a part or all of the organic solvent derived from an oil phase as an evaporation step and a step of naturally evaporating a part or all of the organic solvent in an oil phase in the process of stirring and emulsifying.

The method for evaporating an organic solvent in the evaporation step is not particularly limited. However, for example, at least one of a step of evaporating an organic solvent through heating, a step of being left to stand or continuing gentle stirring after the emulsification, or a step of performing vacuum degassing may be performed.

In the step of evaporating an organic solvent in the present invention, it is preferable to set the concentration of the organic solvent contained in an aqueous solution containing liposomes to less than or equal to 15 mass% within 30 minutes from the start of the step of evaporating an organic solvent.

The liquid temperature in a case of producing the liposome can be appropriately adjusted. The liquid temperature at the time of mixing an oil phase with an aqueous phase is preferably higher than or equal to a phase transition temperature of a lipid to be used. For example, in a case where a lipid having a phase transition temperature of 35°C to 60°C is used, liquid temperature is preferably 40°C to 70°C.

An aqueous solution containing liposomes prepared through the emulsification step is subjected to after-treatment through methods such as centrifugal separation, ultrafiltration, dialysis, gel filtration, and freeze-drying in order to remove components not contained in the liposomes or adjust the concentration or osmotic pressure.

The obtained liposomes may have a uniform particle diameter through a dialysis method, a filtration method, an extrusion treatment, or the like. In the production of a liposome, it is preferable to prepare an empty liposome in a state in which a drug is not loaded without being subjected to extrusion treatment. In addition, in order to separate the drug contained in the liposome from the drug not contained in the liposome, a centrifugal separation method, a dialysis method, a gel filtration method, or the like can be used.

The extrusion treatment means a step of applying a physical shear force by passing liposomes through a filter having pores for atomization.

(Drug Loading Step)

In the drug loading step, a drug is encapsulated in a liposome.

A drug solution is prepared by dissolving the drug in an aqueous medium, and the drug solution and the drug non-encapsulated liposome are mixed with each other to prepare a drug loading solution. Next, dialysis is carried out using a dialysis solution (with an appropriate concentration of sucrose / histidine aqueous solution or the like) to remove the non-encapsulated drug and a solute present in the outer aqueous phase of the drug loading solution, and the outer aqueous phase is replaced with the dialysis solution. In this manner, it is possible to produce a liposome composition in which the drug is encapsulated in the liposome.

(Aseptic Filtration)

In order to make the aqueous solution containing a liposome which is obtained as described above be a liposome composition, it is preferable to carry out aseptic filtration. As a filtration method, unnecessary substances can be removed from an aqueous solution containing liposomes using a hollow fiber membrane, a reverse osmosis membrane, a membrane filter, or the like. The present invention is not particularly limited, the filtration is preferably performed using a filter having a sterilizable pore diameter (preferably a 0.2 $\mu$m filtration sterilization filter). Adsorption or aggregation of liposomes on a filtration sterilization filter may usually occur in the filtration step.

In order to prevent the influence on the average particle diameter due to deformation of liposomes, it is preferable to carry out the aseptic filtration step and the aseptic filling step, to be described below, at a temperature lower than or equal to the phase transition temperature of a lipid forming the liposome. For example, in a case where the phase transition temperature of the lipid is around 50°C, about 0°C to 40°C is preferable and about 5°C to 30°C is more specifically preferable.

(Aseptic filling)

The aqueous solution containing a liposome obtained after the aseptic filtration is preferably subjected to aseptic filling for medical use. A well-known aseptic filling method can be applied. It is possible to prepare a suitable liposome composition for medical use by aseptically filling a container therewith.

[Volume of Tumor]

In the present invention, a tumor can be transplanted into an animal as a model (preferably a mouse or a rat) in order to measure the volume of the tumor. In a case of administering the liposome or liposome composition of the present invention to a subject such as a mammal, it is possible to observe an effect of suppressing growth in the volume of a tumor. The suppression of the growth in the volume of tumor depends on drugs used, a combination of lipids forming a liposome or the like, and an effective amount. The suppression of the growth in the volume of a tumor means at least one whether the growth of a tumor can be suppressed, whether a tumor stops its growth, and whether a tumor substantially or completely regresses.

The volume of a tumor in a mammalian subject can be measured through any arbitrary methods accepted in the field. For example, the volume of a tumor can be evaluated using an equation of $(a \times b^2) \times 0.5$ (where "a" is a maximum diameter and "b" is a length of a minor axis) through measurement using calipers. In addition, the volume of a tumor of a human can be measured through techniques such as image diagnosis such as computed tomography (CT) scanning or magnetic resonance imaging (MRI) scanning.

The liposome and the liposome composition of the present invention can show moderate retentivity in blood and a high treatment index (drug efficacy / toxicity). According to the liposome and the liposome composition of the present invention, it is possible to reduce side effects without reducing the drug efficacy. The liposome and liposome composition of the present invention can be applied to pharmaceutical products, cosmetics, food, and the like. For example, the liposome and liposome composition of the present invention can contain an anticancer agent and can be used as an anticancer agent.

Examples

Hereinafter, the present invention will be described in detail using examples. However, the present invention is not limited to the examples.

The mixing ratio in the solvent composition means a volume ratio. For example, "ethanol / ethyl acetate = 75/25" means 75% ethanol / 25% ethyl acetate by a volume ratio.

In addition, 1 mol/m$^3$=10$^{-3}$ mol/L=1 mmol/L.

In the examples, the following reagents are used.

Dimyristoyl phosphatidylethanolamine (DMPE-PEG2000) to which PEG having a weight-average molecular weight of 2,000 is bonded: SUNBRIGHT PM-020CN (manufactured by NOF CORPORATION)

Dipalmitoyl phosphatidylethanolamine (DPPE-PEG5000) to which PEG having a weight-average molecular weight of 5,000 is bonded: SUNBRIGHT PP-050CN (manufactured by NOF CORPORATION)

Distearoyl phosphatidylethanolamine (DSPE-PEG2000) to which PEG having a weight-average molecular weight of 2,000 is bonded: SUNBRIGHT DSPE-020CN (manufactured by NOF CORPORATION)

Distearoyl phosphatidic acid (DSPA): COATSOME MA-8080LS (manufactured by NOF CORPORATION)

Hydrogenated soybean phosphatidylcholine (HSPC): COATSOME NC21E (manufactured by NOF CORPORATION)

Cholesterol (Chol): Cholesterol HP (manufactured by Dishman)

Dimyristoyl phosphatidylcholine (DMPC): COATSOME MC-4040 (manufactured by NOF CORPORATION)

Doxorubicin hydrochloride: doxorubicin hydrochloride (manufactured by MicroBiopharm Japan Co., Ltd.)

The average carbon chain length of DMPE is 14, the average carbon chain length of DPPE is 16, and the average carbon chain length of DSPE is 18. The average carbon chain length of HSPC is greater than 16 and less than 18.

Example 1

a) Preparation of Oil Phase

DMPE-PEG2000 (component A: a phospholipid to which a hydrophilic polymer is bonded), DSPA (component B: phospholipid having a negative charge within a range of a pH of 6 to 8), HSPC (component C: a phospholipid which is electrically neutral within a range of a pH of 6 to 8), and cholesterol (Chol) were mixed with each other so as to have a molar ratio of 5/5/50/40, 405 ml of an organic solvent (ethanol / ethyl acetate = 75/25) was added thereto so that the concentration of the above-described lipid mixture contained in the oil phase became 51 mg/ml, the mixture was heated to 70°C, and the lipids were dissolved therein to prepare an oil phase.

b) Preparation of Aqueous Phase

26.7 g of ammonium sulfate was dissolved in 1,080 ml of water for injection of Japanese Pharmacopoeia to prepare an aqueous phase.

c) Preparation of Drug Non-Contained Liposome

The aqueous phase was heated to 70°C and the oil phase was added thereto. Then, the aqueous phase and the oil phase were mixed with each other using a rotary stirring emulsifier at a circumferential speed of 30 m/s at 19,000 rpm for 30 minutes. Thereafter, the organic solvent and water were evaporated by supplying nitrogen thereto while heating the mixture to higher than or equal to a phase transition temperature. About 1,000 mL of the organic solvent and the water were evaporated to concentrate the mixture. The outer aqueous phase was dialyzed and replaced with 410 mmol/L sodium chloride as a dialysis solution and was further diluted with a 410 mmol/L dialysis solution so that the lipid mixture became 32 mg/ml to prepare a drug non-contained liposome.

d) Preparation of Drug-Contained Liposome

Drug Loading

1) Preparation of Drug Loading Solution

A 4 mg/mL solution of doxorubicin hydrochloride (manufactured by MicroBiopharm Japan Co., Ltd.) was added to and dissolved in a sodium chloride solution to prepare a drug solution. Subsequently, the drug solution was mixed with a drug non-encapsulated liposome at a volume ratio of 1:1. The mixture was heated at 57°C for 60 minutes to prepare a drug loading solution.

Completion of Liposome Composition through Dialysis

A 275 mmol/L sucrose/10 mmol/L histidine aqueous solution was prepared as a dialysis solution. Dialysis was performed at room temperature using this dialysis solution, each solute and non-encapsulated gemcitabine hydrochloride present in the outer aqueous phase of the drug loading solution were removed, and the outer aqueous phase was replaced with the dialysis solution to obtain a liposome composition in which doxorubicin is encapsulated.

Example 2

A liposome composition containing doxorubicin was obtained in the same manner as in Example 1 except that DMPE-PEG2000 (component A) in a) Preparation of Oil Phase was changed to DPPE-PEG5000 (component A).

Comparative Example 1

A liposome composition containing doxorubicin was obtained in the same manner as in Example 1 except that HSPC (component C), cholesterol, and DSPE-PEG2000 were mixed with each other so as to have a molar ratio of 55/40/5 without adding DMPE-PEG2000 (component A) and DSPA (component B) in a) Preparation of Oil Phase thereto.

Comparative Example 2

A liposome composition containing doxorubicin was obtained in the same manner as in Comparative Example 1 except that HSPC (component C), cholesterol, and DSPE-PEG2000 were mixed with each other so as to have a molar ratio of 59/40/1.

Comparative Example 3

A liposome composition containing doxorubicin was obtained in the same manner as in Comparative Example 1 except that DMPC was mixed with HSPC (component C), cholesterol, and DSPE-PEG2000 so as to have a molar ratio of 41/40/5/14.

Comparative Example 4

A liposome composition containing doxorubicin was obtained in the same manner as in Example 1 except that DMPE-PEG2000 (component A), HSPC (component C), and cholesterol were mixed with each other so as to have a molar ratio of 5/55/40 without adding DSPA (component B) in a) Preparation of Oil Phase thereto.

<Measurement of Average Particle Diameter>

A sample was diluted with an ultrapure water 10 times the volume and the volume average particle diameter was measured as the average particle diameter through a dynamic light scattering method using F-PAR (manufactured by OTSUKA ELECTRONICS Co., LTD.) The results are shown in Table 1.

[Table 1]

|  | Example 1 | Example 2 | Comparative Example 1 | Comparative Example 2 | Comparative Example 3 | Comparative Example 4 |
|---|---|---|---|---|---|---|
| Average particle diameter (nm) | 89 | 98 | 82 | 118 | 76 | 97 |

<Evaluation 1 of Blood Kinetics>

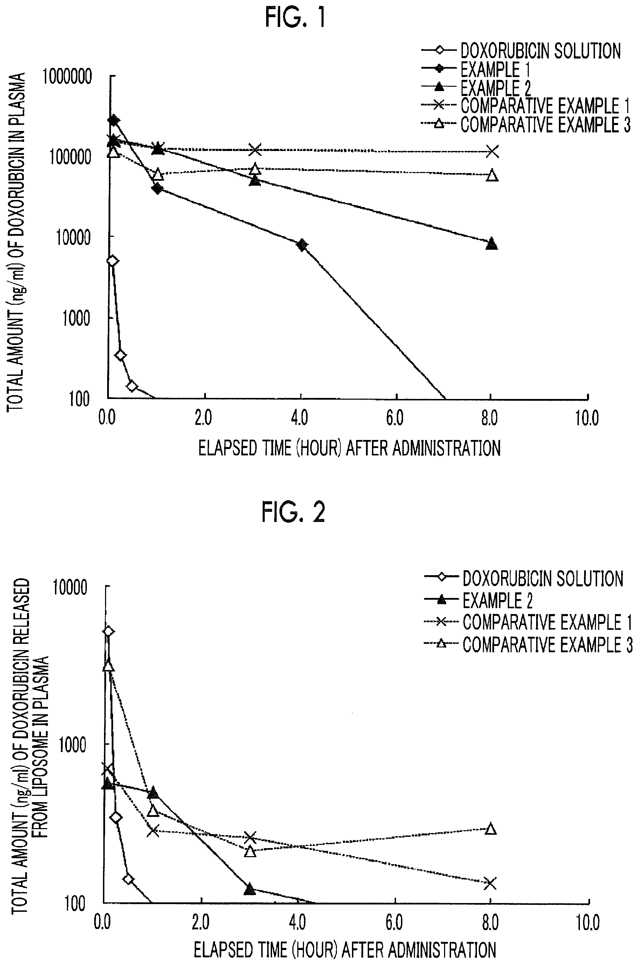

An amount of a drug of 9.4 mass% sucrose aqueous solution (also referred to as a doxorubicin solution) (0.9 mg/ml) of doxorubicin hydrochloride or each 9 mg/kg liposome composition of Example 1 or 2 or Comparative Example 1 or 3 was administered intravenously to 6-week female BALB/c mice under the satiation condition. Blood samples were collected 5 minutes, 15 minutes, 30 minutes, 1 hour, 2 hours, 4 hours, and 8 hours after the administration of the doxorubicin solution and 5 minutes, 1 hour, 3 or 4 hours, and 8 hours after the administration of the liposomes of Examples 1 and 2 and Comparative Examples 1 and 3. The collected blood samples were subjected to centrifugal separation and the concentration of doxorubicin in each plasma sample was quantitatively determined. The results are shown in Fig. 1. In addition, the above-described plasma samples except for Example 1 were subjected to ultrafiltration to quantitatively determine the concentration of doxorubicin in the plasma samples released from the liposomes. The results are shown in Fig. 2.

It can be seen from the results of Fig. 1 that the compositions of Examples 1 and 2 and Comparative Example 3 are liposome compositions in which disappearance of a drug from blood is rapider than that of Comparative Example 1, whereas the retention time of doxorubicin in blood is remarkably prolonged. It can be seen from the results in Fig. 2 that the amount of leakage of doxorubicin from the liposome into blood at the early stage of the administration in Comparative Example 3 is large, whereas the leakage of doxorubicin from the liposomes into blood at the early stage of the administration in Example 2 and Comparative Example 1 can be suppressed.

<Evaluation 2 of Blood Kinetics>

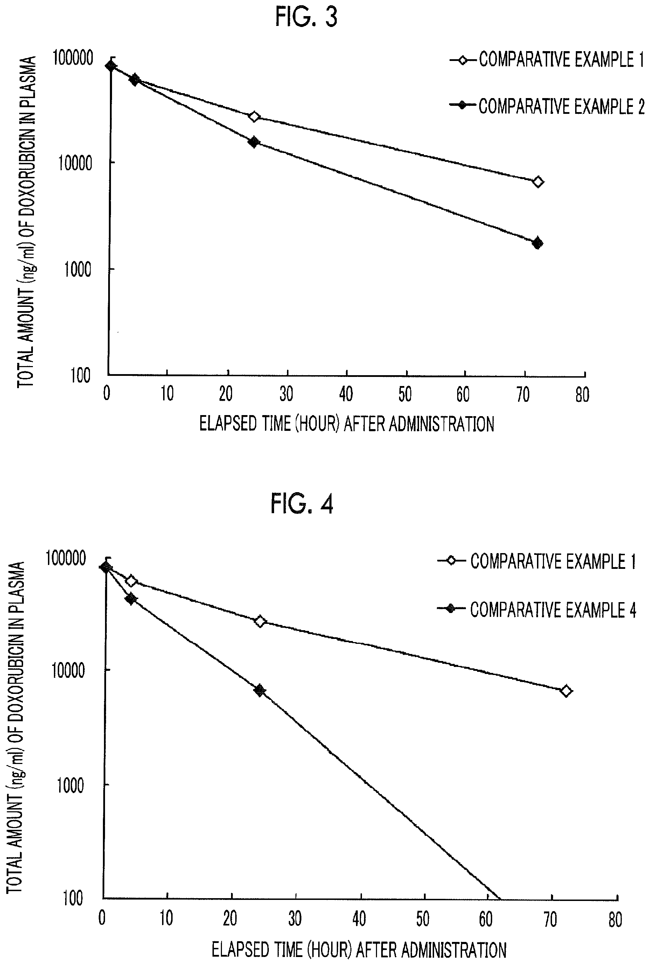

The liposome compositions of Comparative Examples 1 and 2 were administered to 6-week female BALB/c mice under satiation conditions through intravenous injection with a drug amount of 4 mg/kg. Blood samples were taken at 5 minutes, 4 hours, 24 hours, and 72 hours after the administration and subjected to centrifugal separation. The concentration of doxorubicin in plasma was quantitatively determined. The results are shown in Fig. 3.

It can be seen from these results that the composition of Comparative Example 2 is a liposome composition in which disappearance of the drug from blood is rapider than that of Comparative Example 1.

<Evaluation 3 of Blood Kinetics>

The liposome compositions of Comparative Examples 1 and 4 were administered to 6-week female BALB/c mice under satiation conditions through intravenous injection with a drug amount of 4 mg/kg. Blood samples were taken at 5 minutes, 4 hours, 24 hours, and 72 hours after the administration and subjected to centrifugal separation. The concentration of doxorubicin in plasma was quantitatively determined. The results are shown in Fig. 4.

It can be seen from these results that the composition of Comparative Example 4 is a liposome composition in which disappearance of the drug from blood is rapider than that of Comparative Example 1.

<Drug Efficacy Test 1>

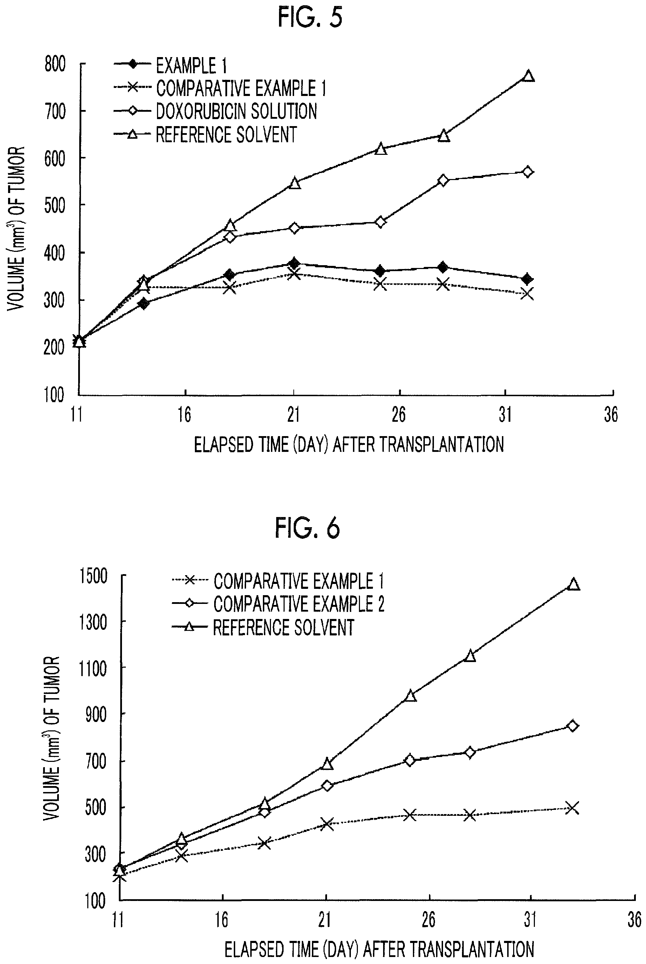

HCT116 cells of a human colon cancer cell strain were transplanted subcutaneously into a BALB/C nude mouse to form subcutaneous tumors. Administration of a 9.4 mass% sucrose aqueous solution of doxorubicin hydrochloride (0.4 mg/ml) of doxorubicin hydrochloride, the liposome compositions (4 mg/kg/week) of Example 1 and Comparative Example 1, and a reference solvent (9.4 mass% sucrose aqueous solution) was started from day 11 after the transplantation. The liposome compositions and the reference solvent were intravenously administered. Once a week was set as a course and in total three courses were performed. The volume of tumors was measured with calipers, and the effect of suppressing the subcutaneous tumors was evaluated. The results are shown in Fig. 5.

It can be seen from these results that the liposome compositions of Example 1 and Comparative Example 1 have high drug efficacy equivalent to the case where doxorubicin hydrochloride was administered.

<Drug Efficacy Test 2>

HCT116 cells of a human colon cancer cell strain were transplanted subcutaneously into a BALB/C nude mouse to form subcutaneous tumors. Administration of the liposome compositions (4 mg/kg/week) of Comparative Examples 1 and 2, and a reference solvent (9.4 mass% sucrose aqueous solution) was started from day 11 after the transplantation. The liposome compositions and the reference solvent were intravenously administered. Once a week was set as a course and in total three courses were performed. The volume of tumors was measured with calipers, and the effect of suppressing the subcutaneous tumors was evaluated. The results are shown in Fig. 6.

It can be seen from these results that the liposome composition of Comparative Example 2 attenuates the drug efficacy compared to the case where the liposome composition of Comparative Example 1 was administered.

<Drug Efficacy Test 3>

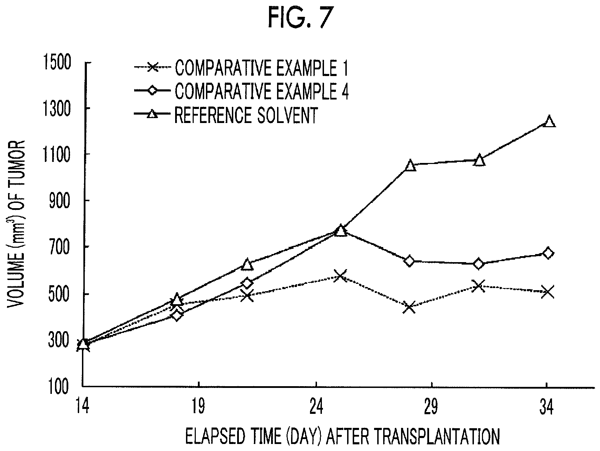

HCT116 cells of a human colon cancer cell strain were transplanted subcutaneously into a BALB/C nude mouse to form subcutaneous tumors. Administration of the liposome compositions (4 mg/kg/week) of Comparative Examples 1 and 4, and a reference solvent (9.4 mass% sucrose aqueous solution) was started from day 14 after the transplantation. The liposome compositions and the reference solvent were intravenously administered. Once a week was set as a course and in total three courses were performed. The volume of tumors was measured with calipers, and the effect of suppressing the subcutaneous tumors was evaluated. The results are shown in Fig. 7.

It can be seen from these results that the liposome compositions of Comparative Examples 1 and 4 have high drug efficacy equivalent to the case where doxorubicin hydrochloride was administered.

<Toxicity Test>

The liposome compositions of Example 1 and 2 and Comparative Examples 1, 2, 3, and 4 and a reference solvent (9.4 mass% sucrose aqueous solution) were administered to BALB/C female mice at a dosage of 9 mg/kg 6 times (twice a week) as a drug. The general state was observed in order to evaluate hand-foot syndrome. The swelling on feet of surviving mice was observed to evaluate the presence or absence of swelling. In addition, necropsy was performed 1 week after the last administration and the degree of reddening of the skin was evaluated. The results are shown in Table 2.

[Table 2]

| | | Example 1 | Example 2 | Comparative Example 1 | Comparative Example 2 | Comparative Example 3 | Comparative Example 4 | Reference solvent |
|---|---|---|---|---|---|---|---|---|
| Swelling of feet* | Incidence | 20 | 2C | 75 | 4C | | 100 | 0 |
| Reddening of skin ** | - | 60 | 80 | 0 | 0 | (Evaluation cannot be performed due to deterioration of general state and death) | 0 | 100 |
| | + | 40 | 20 | 0 | 0 | | 0 | 0 |
| | 2+ | 0 | 0 | 75 | 80 | | 33 | 0 |
| | 3+ | 0 | 0 | 25 | 0 | | 67 | 0 |

*:

*: Incidence (%) of swelling on feet = (number of mice with swelling observed even with one inner finger of limb) / (number of mice in whole group) × 100

**:

**: Incidence (%) of reddening of skin = (number of mice at each score) / (number of mice in whole group) × 100

-: 0 to 2 red spots with a diameter of less than or equal to 1 mm, or a plurality of fine red points

+: 1 or 2 red spots with a diameter of greater than or equal to 1 mm and a plurality of fine red spots, or red spots with a diameter of greater than or equal to 5 mm 2+: 3 or more red spots with a diameter of greater than or equal to 1 mm, or red spots with a diameter of greater than or equal to 10 mm 3+: a plurality of red spots with a diameter of greater than or equal to 1 mm and a plurality of red spots with a diameter of greater than or equal to 5 mm

It can be seen from the results that the hand-foot syndromes of Examples 1 and 2 and Comparative Example 2 are reduced compared to that of Comparative Example 1. In Comparative Example 3, deterioration of the general condition or death of animals was observed during the evaluation, which shows a result of strong toxicity. Regarding Comparative Example 4, death of some animals was observed and the hand-foot syndrome also became abhorrent.

<Summary of Test Results>

The test results are summarized in Table 3. It was found that Examples 1 and 2 of the present invention can provide liposome formulations which exhibit a strong effect of suppressing a tumor, reduce the hand-foot syndrome, have no lethal change observed in mice, and have a high treatment index.

In Comparative Example 2, excessive retentivity in blood shown by Comparative Example 1 was decreased by reducing the PEG modification amount, and the hand-foot syndrome was reduced. However, it was presumed that the drug efficacy would be reduced without sufficient accumulation on tumors since liposome particles disappear from blood immediately after the administration.

In Comparative Example 3, the substance permeability of a liposome membrane was promoted using DMPC with a shorter alkyl chain length and a lower melting point than those of HSPC, thereby improving the leakage immediately after the administration. It was estimated that this increased the exposure amount of doxorubicin to the organs of the whole body and the toxicity became abhorrent.

In Comparative Example 4, the liposome surface was PEG-modified with DMPE-PEG2000 having a shorter average alkyl chain than that of HSPC. Immediately after the administration, both disappearance of liposome particles and release of doxorubicin were sufficiently suppressed equivalently to Comparative Example 1, and sufficient accumulation in tumors was achieved while suppressing cardiotoxicity. The drug efficacy equivalent to Comparative Example 1 was shown in Comparative Example 4. In this lipid, it is estimated that the hand-foot syndrome is deteriorated such that the PEG lipid is desorbed after a certain period of time after the administration in blood, the retention time in blood becomes short, negative charges included in the PEG lipid also disappear at the same time, and transition to a specific organ is promoted.

In Examples 1 and 2, it was estimated that the hand-foot syndrome could be reduced such that both disappearance of liposome particles and release of doxorubicin were sufficiently suppressed equivalently to Comparative Example 1 immediately after the administration, sufficient accumulation in tumors was achieved while suppressing cardiotoxicity, the PEG lipid was desorbed from liposome particles after a certain period of time, the accumulation in the skin was suppressed, negative charges which included in the PEG lipid and disappeared were charged by electric charges included in negatively charged lipids which had not been desorbed, and transition to a specific organ was suppressed.

[Table 3]

| | Lipid composition | Blood kinetics | Drug efficacy T/C (%) * | Drug efficacy | Incidence (%) of swelling on feet in hand-foot syndrome ** | Hand-foot syndrome | Toxicity in whole body (death) |
|---|---|---|---|---|---|---|---|
| Example 1 | DMPE-PEG2000, DSPA, HSPC, Chol=5/5/50/40 | More prolonged retention time than that of doxorubicin and rapider disappearance of drug than that of Comparative Example 1 Leakage of drug from liposome at early stage of administration can be suppressed | 44 | Effect of suppressing growth of tumor which was equivalent to Comparative Example 1 was shown | 20 | Lower than Comparative Example 1 | Did not occur |
| Example 2 | DPPE-PEG5000, DSPA, HSPC, Chol=5/5/50/40 | | - | - | 20 | Lower than Comparative Example 1 | Did not occur |
| Comparative Example 1 | DSPE-PEG2000, HSPC, Chol=5/55/40 | Prolonged retention time in blood | 40 | High effect of suppressing growth of tumor was shown | 75 | Became highly toxic | Did not occur |
| Comparative Example 2 | DSPE-PEG2000, HSPC Chol=1/59/40 | More prolonged retention time than that of doxorubicin and rapider disappearance of drug than that of Comparative Example 1 | 58 | Effect of suppressing tumor decreased compared to that of Comparative Example 1 | 40 | Lower than Comparative Example 1 | Did not occur |
| Comparative Example 3 | DSPE-PEG2000, HSPC, DMPC, Chol=5/41/14/40 | More prolonged retention time than that of doxorubicin and rapider disappearance of drug than that of Comparative Example 1 Drug leaks out from liposome at early stage of administration and final concentration was higher than that of Comparative Example 1 | | - | Evaluation cannot be performed because of death of animal due to strong toxicity | Evaluation cannot be performed because of death of animal due to strong toxicity | Dead animal was recognized due to strong toxicity |

(continued)

| | Lipid composition | Blood kinetics | Drug efficacy T/C (%) * | Drug efficacy | Incidence (%) of swelling on feet in hand-foot syndrome ** | Hand-foot syndrome | Toxicity in whole body (death) |
|---|---|---|---|---|---|---|---|
| Comparative Example 4 | DMPE-PEG2000, HSPC, Chol=5/55/40 | More prolonged retention time than that of doxorubicin and rapider disappearance of drug than that of Comparative Example 1 Leakage of drug from liposome at early stage of administration can be suppressed | 54 | Effect of suppressing growth of tumor which was equivalent to Comparative Example 1 was shown | 100 | Deteriorated compared to Comparative Example 1 | Dead animal was recognized during evaluation of toxicity |
| Doxorubicin solution | None | Significantly rapid disappearance of drug Final concentration was high | 73 | Although there was effect of suppressing growth of tumor, effect was lower than that of Comparative Example 1 | 0 | Did not occur | Became highly toxic |

*: Drug efficacy T/C (%) = (volume of tumor at test end date of drug-administered group) / (volume of tumor at test end date of reference solvent group) × 100

**: Incidence (%) of swelling on feet = (number of mice with swelling observed even with one inner finger of limb) / (number of mice in whole group) × 100

## Claims

1. A liposome containing at least a phospholipid to which a hydrophilic polymer is bonded, which is one or more types selected from dipalmitoyl phosphatidylethanolamine to which PEG having a weight-average molecular weight of 5,000 is bonded (DPPE-PEG5000) or dimyristoyl phosphatidylethanolamine to which PEG having a weight-average molecular weight of 2,000 is bonded (DMPE-PEG2000), a phospholipid having a negative charge within a range of a pH of 6 to 8, which is one or more types selected from the group consisting of phosphatidic acid, phosphatidylglycerol, phosphatidylserine, and phosphatidylinositol, and a phospholipid which is electrically neutral within a range of a pH of 6 to 8, which is phosphatidylcholine having an average carbon chain length of the phospholipid of 16 to 18, wherein an average carbon chain length of the phospholipid to which a hydrophilic polymer is bonded is shorter than an average carbon chain length of the phospholipid which is electrically neutral within a range of a pH of 6 to 8.

2. The liposome according to claim 1,
wherein a content ratio of the phospholipid to which a hydrophilic polymer is bonded with respect to all lipids forming a liposome membrane is 1 to 20 mol%, a content ratio of the phospholipid having a negative charge within a range of a pH of 6 to 8 with respect to all the lipids forming the liposome membrane is 1 to 20 mol%, and a content ratio of the phospholipid which is electrically neutral within a range of a pH of 6 to 8 with respect to all the lipids forming the liposome membrane is 30 to 80 mol%.

3. The liposome according to any one of claims 1 to 2, further comprising:
one or more other types of lipids in addition to the phospholipid to which a hydrophilic polymer is bonded, the phospholipid having a negative charge within a range of a pH of 6 to 8, and the phospholipid which is electrically neutral within a range of a pH of 6 to 8.

4. The liposome according to any one of claims 1 to 3,
wherein the other types of the lipids are cholesterols.

5. The liposome according to any one of claims 1 to 4 which is dispersed in a medium which disperses the liposome.

6. A liposome composition comprising:

   the liposome according to any one of claims 1 to 5; and
   a drug contained in the liposome.

7. The liposome composition according to claim 6,
wherein the drug has a distribution coefficient LogD of less than or equal to 1.

## Patentansprüche

1. Liposom, enthaltend mindestens ein Phospholipid, an das ein hydrophiles Polymer gebunden ist, bei dem es sich um eine oder mehrere Arten handelt, ausgewählt aus Dipalmitoylphosphatidylethanolamin, an das PEG mit einem gewichtsmittleren Molekulargewicht von 5.000 gebunden ist (DPPE-PEG5000) oder Dimyristoylphosphatidylethanolamin, an das PEG mit einem gewichtsmittleren Molekulargewicht von 2.000 gebunden ist (DMPE-PEG2000), ein Phospholipid mit einer negativen Ladung innerhalb eines pH-Bereichs von 6 bis 8, bei dem es sich um eine oder mehrere Arten handelt, ausgewählt aus der Gruppe bestehend aus Phosphatidsäure, Phosphatidylglycerol, Phosphatidylserin und Phosphatidylinositol, und ein Phospholipid, das innerhalb eines pH-Bereichs von 6 bis 8 elektrisch neutral ist, bei dem es sich um Phosphatidylcholin mit einer durchschnittlichen Kohlenstoffkettenlänge des Phospholipids von 16 bis 18 handelt, worin die durchschnittliche Kohlenstoffkettenlänge des Phospholipids, an das ein hydrophiles Polymer gebunden ist, kürzer ist als die durchschnittliche Kohlenstoffkettenlänge des Phospholipids, das innerhalb eines pH-Bereichs von 6 bis 8 elektrisch neutral ist.

2. Liposom gemäß Anspruch 1, worin ein Gehaltsverhältnis des Phospholipids, an das ein hydrophiles Polymer gebunden ist, in Bezug auf alle Lipide, die eine Liposomenmembran bilden, 1 bis 20 Mol-% beträgt, ein Gehaltsverhältnis des Phospholipids mit einer negativen Ladung innerhalb eines pH-Bereichs von 6 bis 8 in Bezug auf alle Lipide, die eine Liposomenmembran bilden, 1 bis 20 Mol-% beträgt und ein Gehaltsverhältnis des Phospholipids, das innerhalb eines pH-Bereichs von 6 bis 8 elektrisch neutral ist, in Bezug auf alle Lipide, die eine Liposomenmembran bilden, 30 bis 80 Mol-% beträgt.

**3.** Liposom gemäß einem beliebigen der Ansprüche 1 bis 2, ferner umfassend:

eine oder mehrere Arten von Lipiden zusätzlich zu dem Phospholipid, an das ein hydrophiles Polymer gebunden ist, dem Phospholipid mit einer negativen Ladung innerhalb eines pH-Bereichs von 6 bis 8, und dem Phospholipid, das innerhalb eines pH-Bereichs von 6 bis 8 elektrisch neutral ist.

**4.** Liposom gemäß einem beliebigen der Ansprüche 1 bis 3, worin die anderen Arten von Lipiden Cholesterole sind.

**5.** Liposom gemäß einem beliebigen der Ansprüche 1 bis 4, das in einem Medium dispergiert ist, welches das Liposom dispergiert.

**6.** Liposomzusammensetzung, umfassend:
das Liposom gemäß einem beliebigen der Ansprüche 1 bis 5 und ein in dem Liposom enthaltenen Arzneistoff.

**7.** Liposomzusammensetzung gemäß Anspruch 6, worin der Arzneistoff einen Verteilungskoeffizienten LogD von weniger als oder gleich 1 aufweist.


**Revendications**

**1.** Liposome contenant au moins un phospholipide auquel un polymère hydrophile est lié, qui est un ou plusieurs types sélectionnés parmi la dipalmitoyl phosphatidyléthanolamine à laquelle du PEG présentant un poids moléculaire moyen en poids de 5 000 est lié (DPPE-PEG5000) ; ou la dimyristoyl phosphatidyléthanolamine à laquelle du PEG présentant un poids moléculaire moyen en poids de 2000 est lié (DMPE-PEG2000), un phospholipide présentant une charge négative au sein d'une plage de pH de 6 à 8, qui est un ou plusieurs types sélectionnés parmi le groupe constitué par l'acide phosphatidique, le phosphatidylglycérol, la phosphatidylsérine et le phosphatidylinositol, et un phospholipide qui est électriquement neutre au sein d'une plage de pH de 6 à 8, qui est une phosphatidylcholine présentant une longueur de chaîne carbonée moyenne du phospholipide de 16 à 18, dans lequel une longueur de chaîne carbonée moyenne du phospholipide à laquelle un polymère hydrophile est lié est plus courte qu'une longueur de chaîne carbonée moyenne du phospholipide qui est électriquement neutre au sein d'une plage de pH de 6 à 8.

**2.** Liposome selon la revendication 1, dans lequel un rapport de teneur du phospholipide auquel un polymère hydrophile est lié par rapport à tous les lipides formant une membrane de liposome est de 1 à 20 % en moles, un rapport de teneur du phospholipide présentant une charge négative au sein d'une plage de pH de 6 à 8 par rapport à tous les lipides formant la membrane de liposome est de 1 à 20 % en moles et un rapport de teneur du phospholipide qui est électriquement neutre au sein d'une plage de pH de 6 à 8 par rapport à tous les lipides formant la membrane de liposome est de 30 à 80 % en moles.

**3.** Liposome selon l'une quelconque des revendications 1 à 2, comprenant en outre :
un ou plusieurs autres types de lipides en plus du phospholipide auquel un polymère hydrophile est lié, le phospholipide présentant une charge négative au sein d'une plage de pH de 6 à 8, et le phospholipide qui est électriquement neutre au sein d'une plage de pH de 6 à 8.

**4.** Liposome selon l'une quelconque des revendications 1 à 3, dans lequel les autres types de lipides sont les cholestérols.

**5.** Liposome selon l'une quelconque des revendications 1 à 4 qui est dispersé dans un milieu qui disperse le liposome.

**6.** Composition de liposome comprenant :

le liposome selon l'une quelconque des revendications 1 à 5 ; et
un médicament contenu dans le liposome.

**7.** Composition de liposome selon la revendication 6, dans laquelle le médicament présente un coefficient de répartition LogD inférieur ou égal à 1.

# FIG. 1

# FIG. 2

## FIG. 3

## FIG. 4

## FIG. 5

## FIG. 6

# FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9610392 A

- JP 2889549 B

**Non-patent literature cited in the description**

- *Cancer Research,* 1986, vol. 46, 6387-6392

- *Arch Dermatol.,* 2000, vol. 136, 1475-1780